(19) Europäisches Patentamt
European Patent Office
Office européen des brevets

(11) Veröffentlichungsnummer: **0 009 697**
**B1**

(12) # EUROPÄISCHE PATENTSCHRIFT

(45) Veröffentlichungstag der Patentschrift: **23.09.81**

(51) Int. Cl.³: **C 25 B 3/02,** C 07 C 125/065, C 07 D 263/26

(21) Anmeldenummer: **79103465.5**

(22) Anmeldetag: **17.09.79**

(54) **Verfahren zur Herstellung von N-(Alpha-Methoxy-alkyl)-urethanen und neue N-(Alpha-methoxy-alkyl)-urethane.**

(30) Priorität: **30.09.78 DE 2842760**

(43) Veröffentlichungstag der Anmeldung: **16.04.80 Patentblatt 80/8**

(45) Bekanntmachung des Hinweises auf die Patenterteilung: **23.09.81 Patentblatt 81/38**

(84) Benannte Vertragsstaaten: **CH DE FR GB**

(56) Entgegenhaltungen:
**DE-A-2 113 338**
**FR-A-2 296 614**
**FR-A-2 322 863**
**JOURNAL OF THE AMERICAN CHEMICAL SOCIETY, vol. 97, 23th July 1975, Seiten 4264—4268**
**T. SHONO et al.: »Anodic Oxidation of Carbamates«**

(73) Patentinhaber: **BAYER AG, Zentralbereich Patente, Marken und Lizenzen, D-5090 Leverkusen 1, Bayerwerk (DE)**

(72) Erfinder: **Buhmann, Hans Rolf, Dr., Richrather Strasse 92, D-4018 Langenfeld (DE)**
Erfinder: **Arlt, Dieter, Dr., Rybniker Strasse 2, D-5000 Köln 80 (DE)**
Erfinder: **Müller, Hanns Peter, Dr., St. Pankratius-Strasse 2, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 009 697 B1

BUNDESDRUCKEREI BERLIN

Verfahren zur Herstellung von N-($\alpha$-Methoxy-alkyl)-urethanen und neue N-($\alpha$-Methoxy-alkyl)-urethane

Die Erfindung betrifft ein Verfahren zur Herstellung von N-($\alpha$-Methoxy-alkyl)-urethanen durch elektrochemische Oxidation von N-Alkyl-urethanen in methanolischer Lösung und neue N-($\alpha$-Methoxy-alkyl)-urethane.

Es ist bekannt, O-Methyl-N-alkyl-urethane und O-Methyl-N,N-dialkyl-urethane durch elektrochemische Oxidation in methanolischer Lösung in N-($\alpha$-Methoxy-alkyl)-urethane bzw. N-($\alpha$-Methoxy-alkyl)-N-alkyl-urethane zu überführen (J. Am. Chem. Soc. 97, 4264 (1975)).

Das genannte Verfahren liefert die gewünschten Urethane oft in nur geringen Ausbeuten, wobei in vielen Fällen schwer abtrennbare Nebenprodukte gebildet werden. Insbesondere N-monoalkylierte Urethane durch Abspaltung eines Alkylrestes aus N,N-dialkylierten Urethanen.

Aus der FR-A-2 322 863 ist es bekannt, N-acylierte cyclische Amine in $\alpha$- oder $\alpha,\alpha'$-Stellung mit $C_1-C_4$-Alkoholen elektrochemisch zu oxidieren, wobei Temperaturen von $-10$ bis $+100°C$, eine Strommenge von 2 bis 2,5 Faraday pro Mol Substrat und eine Stromdichte von 1 bis 50 $A/dm^2$ angewendet werden.

Es wurde ein Verfahren zur Herstellung von am Stickstoff $\alpha$-Methoxy-alkylierten Urethanen durch elektrochemische Oxidation von am Stickstoff alkylierten Urethanen in methanolischer Lösung in Gegenwart eines Leitsalzes bei einer Temperatur unter bis weit über 0°C, einer Strommenge von mindestens 2 Faraday pro Mol eingeführte Methoxygruppe und einer Stromdichte bis zu 50 $A/dm^2$ gefunden, das dadurch gekennzeichnet ist, daß man die Elektrolyse im Temperaturbereich von $-20$ bis $+65°C$ bei einer Stromdichte von 0,1 bis 50 $A/dm^2$ durchführt, wobei die Stromdichte im Verlauf der Elektrolyse erniedrigt wird und daß man gegebenenfalls in Gegenwart einer basischen Verbindung arbeitet.

In der elektrochemischen Oxidation können Urethane der Formel (I)

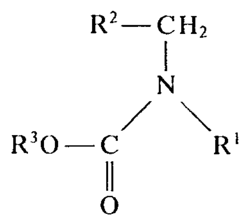

(I)

in der

- $R^1$ für Wasserstoff, Alkyl, Alkenyl, $-CH_2-OCH_3$, $-CH(Alkyl)-OCH_3$ oder $-CH(Alkenyl)-OCH_3$ steht,
- $R^2$ Wasserstoff, Alkyl oder Alkenyl bedeutet und
- $R^3$ für Alkyl, Alkenyl oder $\omega$-Hydroxy-alkyl steht,

wobei außerdem $R^1$ und $R^2$ gemeinsam eine Alkylengruppe darstellen können, die mit den Atomen, die zwischen ihnen stehen, einen Ring bildet, und wobei außerdem auch $R^2$ und $R^3$ gemeinsam eine Alkylengruppe darstellen können, die mit den Atomen, die zwischen ihnen stehen, einen Ring bildet, eingesetzt werden.

Als Alkyl ($R^1$ bis $R^3$) kommt ein geradkettiger oder verzweigter aliphatischer Kohlenwasserstoffrest mit 1 bis 12 Kohlenstoffatomen in Betracht, beispielsweise Methyl, Äthyl, Propyl, Isopropyl, Butyl, Isobutyl, Pentyl, Isopentyl, Hexyl, Isohexyl, Octyl, Isooctyl, Decyl, Isodecyl oder Isododecyl. Bevorzugtes Alkyl ($R^1$ bis $R^3$) sind aliphatische Kohlenwasserstoffreste mit 1 bis 8 Kohlenstoffatomen, beispielsweise Methyl, Äthyl, Propyl, Isopropyl, Butyl, Isobutyl, Hexyl, Isohexyl, Octyl oder Isooctyl. Ganz besonders bevorzugt als Alkyl ($R^1$ bis $R^3$ sind aliphatische Kohlenwasserstoffreste mit 1 bis 4 Kohlenstoffatomen, beispielsweise Methyl, Äthyl, Propyl, Isopropyl, Butyl oder Isobutyl.

Als Alkenyl ($R^1$ bis $R^3$) kommen geradkettige oder verzweigte ungesättigte aliphatische Kohlenwasserstoffreste mit 2 bis 6 Kohlenstoffatomen, bevorzugt mit 2 bis 4 Kohlenstoffatomen, in Betracht, beispielsweise Vinyl, Propenyl, Allyl, Butenyl, Pentenyl oder Hexenyl.

Als $\omega$-Hydroxy-alkyl ($R^3$) kommen geradkettige oder verzweigte aliphatische Kohlenwasserstoffreste mit 1 bis 6 Kohlenstoffatomen, die eine endständige Hydroxygruppe tragen, in Betracht, beispielsweise Hydroxymethyl, 2-Hydroxy-äthyl, 3-Hydroxypropyl, 2-Hydroxy-1-methyl-äthyl, $\omega$-Hydroxy-butyl, $\omega$-Hydroxy-isobutyl, $\omega$-Hydroxyhexyl oder $\omega$-Hydroxy-isohexyl.

Für den Fall, daß $R^1$ und $R^2$ gemeinsam eine Alkylengruppe darstellen, die mit den Atomen, die zwischen ihnen stehen, einen Ring bildet, kommt eine Alkylengruppe mit 3 bis 5 Kohlenstoffatomen in Betracht, beispielsweise Trimethylen, Tetramethylen oder Pentamethylen. In der Alkylengruppe kann weiterhin eine $CH_2$-Gruppe durch ein Sauerstoffatom ersetzt sein.

Für den Fall, daß $R^1$ und $R^3$ gemeinsam eine Alkylengruppe darstellen, die mit den Atomen, die

zwischen ihnen stehen, einen Ring bilden, kommt eine Alkylengruppe mit 1 bis 3 Kohlenstoffatomen in Betracht, beispielsweise Methylen, Äthylen oder Trimethylen.

Als Ausgangsstoff für das erfindungsgemäße Verfahren seien beispielsweise die folgenden Urethane genannt: O-Methyl-N-methyl-urethan, O-Methyl-N-äthyl-urethan, O-Methyl-N-propylurethan, O-Methyl-N-butyl-urethan, O-Methyl-N,N-dimethyl-urethan, O-Methyl-N,N-diäthyl-urethan, O-Äthyl-N-methyl-urethan, O-Äthyl-N-äthyl-urethan, O-(2-Äthyl-hexyl)-N-methyl-urethan, O-(2-Äthyl-hexyl)-N-äthyl-urethan, O-(2-Hydroxyäthyl)-N-methyl-urethan, O-(2-Hydroxy-äthyl)-N-äthyl-urethan, O-(2-Hydroxyäthyl)-N,N-dimethyl-urethan, O-(4-Hydroxybutyl)-N-methyl-urethan, O-(4-Hydroxybutyl)-N-äthyl-urethan, O-(2-Propenyl)-N-methyl-urethan, O-(2-Propenyl)-N-äthyl-urethan, Oxazolidinon(2).

Solche am Stickstoff alkylierten Urethane sind bekannt und können beispielsweise aus Chlorameisensäureestern und Aminen (Houben-Weyl, Methoden der organischen Chemie, Band VIII, Seite 138, Georg Thieme Verlag Stuttgart, 1952) oder aus Isocyanaten und Alkoholen (ebenda, Seite 141) hergestellt werden.

Die Konzentration der Urethane in der methanolischen Lösung beträgt für das erfindungsgemäße Verfahren 1 bis 50 Gew.-%. Bevorzugt ist der Konzentrationsbereich von 10 bis 30 Gew.-%.

Die methanolische Lösung des Urethans für das erfindungsgemäße Verfahren enthält ein Leitsatz in einer Konzentration von 0,01 bis 2 Mol pro Liter, bevorzugt in einer Konzentration von 0,05 bis 0,5 Mol pro Liter. Die Verwendung von Leitsalzen für organische elektrochemische Prozesse sind dem Fachmann bekannt. Beispielsweise seien folgende Leitsalze genannt: Die Alkali-, Erdalkali- oder Tetraalkylammoniumsalze der Perchlorsäure, des Tetrafluorborwasserstoffs, der Hexafluorphosphorsäure, des Fluorwasserstoffs, der Salpetersäure oder der p-Toluolsulfonsäure. Im erfindungsgemäßen Verfahren wird als Leitsalz bevorzugt Tetraäthylammonium-tetrafluorborat eingesetzt. Bei der Aufarbeitung des Reaktionsgemisches fällt dieses bevorzugt eingesetzte Leitsalz nach dem Abdestillieren des Methanols im allgemeinen als Feststoff aus und kann ohne weitere Reinigung wieder im Verfahren eingesetzt werden.

Das erfindungsgemäße Verfahren wird im Temperaturbereich von −20 bis +65°C, bevorzugt im Temperaturbereich von +10 bis +40°C, durchgeführt.

Die elektrochemische Oxidation der oben beschriebenen Urethane im erfindungsgemäßen Verfahren ist mit Ausnahme der Leitsalze grundsätzlich ohne weitere Hilfsstoffe durchführbar. Da jedoch einzelne der erfindungsgemäß herstellbaren am Stickstoff $\alpha$-methoxyalkylierten Urethane säueremfindlich sind und ein Absinken des pH-Wertes im Reaktionsgemisch während der Elektrolyse durch die Entstehung kleiner Mengen von unerwünschten Nebenprodukten nicht immer auszuschließen ist, ist es zweckmäßig, dem Elektrolyten 0,01 bis 2 Gew.-%, bevorzugt 0,05 bis 1 Gew.-% einer unter den Reaktionsbedingungen schwer oxidierbaren Base zuzusetzen, um ein Absinken des pH-Wertes auf Werte kleiner als 7 zu verhindern. Als Beispiele für solche schwer oxidierbaren Basen seien beispielsweise Collidin und 2,6-Lutidin genannt. Es kann jedoch auch zweckmäßig sein, die Elektrolyse ohne den Zusatz einer Base durchzuführen und erst bei der Aufarbeitung säureempfindlicher Reaktionsprodukte, beispielsweise durch destillative Trennung, den pH-Wert auf Werte von 7 oder höher einzustellen.

Die erfindungsgemäße elektrochemische Reaktion kann in einer ungeteilten oder in einer durch ein übliches Diaphragma in einen Anoden- und einen Kathodenraum geteilten Elektrolysezelle durchgeführt werden. Hierbei ist eine in der Konstruktion und im Betrieb weniger aufwendige ungeteilte Elektrolysezelle ohne Nachteil für das Verfahren.

Im erfindungsgemäßen Verfahren wird als Elektrolyseabgas reiner Wasserstoff gebildet, so daß außer einer Spülung der Apparatur mit Inertgas vor Inbetriebnahme keine zusätzlichen Maßnahmen zum Schutz vor einer Knallgasbildung notwendig sind.

Die Zellenkonstruktion ist für das erfindungsgemäße Verfahren nicht kritisch, so lange gewährleistet ist, daß die Reaktionswärme und der gebildete Wasserstoff abgeführt werden können. Beispielsweise sind Plattenzellen mit einem Elektrodenabstand von 1 bis 5 mm, durch die der Elektrolyt parallel zu den Elektroden gepumpt wird, geeignet.

Als Material für die Kathoden kommt beispielsweise Kupfer, Nickel, Stahl, Platin oder Graphit, in bevorzugter Weise Stahl, in Frage.

Das Material der Anoden muß stabil gegenüber den Reaktionsbedingungen des erfindungsgemäßen Verfahrens sein. Solche Materialien sind beispielsweise Graphit, Bleidioxid, ein Metall der Platin-Gruppe, eine Legierung aus Metallen der Platin-Gruppe oder Metalle, die einen Überzug aus einem Platinmetall tragen. Bevorzugt wird Graphit, Platin oder platiniertes Titan als Anodenmaterial verwendet.

Die Formgebung der Elektroden ist für das erfindungsgemäße Verfahren nicht kritisch. Beispielsweise können Platten oder Netze aus den o. g. Elektrodenmaterialien eingesetzt werden, denen der Strom in einer dem Fachmann bekannten Schaltung zugeleitet werden kann.

Wird das erfindungsgemäße Verfahren in einer geteilten Zelle durchgeführt, soll der Elektrolyt im Anoden- und Kathodenraum etwa die gleiche Zusammensetzung haben. Das für die Bereitung des Elektrolyten benutzte Methanol muß nicht hochgradig rein sein. Beispielsweise braucht kein analysenreines Methanol verwendet zu werden. Das erfindungsgemäße Verfahren ist vielmehr auch

mit Methanol von technischer Qualität, das beispielsweise einen geringen Wassergehalt hat, durchzuführen.

Die erforderliche Strommenge beträgt 2 Faraday pro Mol eingeführte Methoxygruppe. Bei Anwendung einer geringeren Strommenge verläuft die Reaktion der Einführung einer Methoxygruppe unvollständig, so daß Produktgemische auftreten können, die eine aufwendige Aufarbeitung erforderlich machen. Daher ist es im erfindungsgemäßen Verfahren zur Erreichung einer möglichst hohen Ausbeute erforderlich, eine Strommenge von mindestens 2 Faraday pro Mol eingeführte Methoxygruppe anzuwenden.

Ein weitgehend vollständiger Umsatz der Ausgangsverbindung vereinfacht die Aufarbeitung, da nur wenig unumgesetztes Ausgangsprodukt abzutrennen ist. Dagegen steigt nach vollständigem Umsatz der Ausgangsverbindung die Gefahr, daß das Reaktionsprodukt in unerwünschter Weise weiter oxidiert wird. Diese unerwünschte Weiteroxidation kann durch eine Erniedrigung der Stromdichte unterdrückt werden. Bei der Anwendung einer niedrigen Stromdichte während des gesamten Elektrolysevorgangs wird jedoch die Reaktionsdauer verlängert.

Es ist daher ein Merkmal des erfindungsgemäßen Verfahrens, daß die Stromdichte im Verlauf der Elektrolyse erniedrigt wird. Bei der diskontinuierlichen Durchführung des erfindungsgemäßen Verfahrens kann man hierzu so verfahren, daß die anfänglich gewählte Stromdichte im Verlaufe der Elektrolyse kontinuierlich oder in mehreren Stufen, bevorzugt in mehreren Stufen, erniedrigt wird, bis die Reaktion nach Aufnahme der gewünschten Strommenge abgebrochen werden kann. Bei kontinuierlicher Durchführung des erfindungsgemäßen Verfahrens kann eine Kaskadenschaltung mehrerer Elektrolysezellen angewendet werden, wobei in jeder Elektrolysezelle die Stromdichte geringer wird als in der vorangegangenen.

Die Stromdichten werden im Bereich von 0,1 bis 50 $A/dm^2$, bevorzugt im Bereich von 1 bis 20 $A/dm^2$ gewählt.

Die Zellenspannung stellt sich in Abhängigkeit von der Zusammensetzung des Elektrolyten, der Temperatur und der Geometrie der Elektrolysezelle ein. Sie nimmt Werte von 4 bis 12 V pro Einzelzelle an.

Das erfindungsgemäße Verfahren kann am Beispiel der Oxidation von O-Methyl-N-methyl-urethan zu O-Methyl-N-methoxymethyl-urethan anhand der folgenden Formelgleichungen verdeutlicht werden:

Anodenreaktion:

$$CH_3O-CO-NHCH_3 + CH_3OH \longrightarrow CH_3O-CO-NH-CH_2OCH_3 + 2H^+ + 2e^-$$

Kathodenreaktion:

$$2H^+ + 2e^- \longrightarrow H_2$$

Gesamtreaktion:

$$CH_3O-CO-NHCH_3 + CH_3OH \longrightarrow CH_3O-CO-NH-CH_2OCH_3 + H_2$$

Das erfindungsgemäße Verfahren kann beispielsweise wie folgt durchgeführt werden:

Der Elektrolyt wird durch Auflösen eines am Stickstoff alkylierten Urethans, des Leitsalzes und gegebenenfalls der basischen Verbindung in Methanol hergestellt und elektrolysiert, wobei die Stromdichte im Verlauf der Elektrolyse erniedrigt wird. Die Elektrolyseprodukte werden nach dem Abdestillieren des Methanols und dem Abfiltrieren des kristallin ausgefallenen Leitsalzes durch Destillation oder Umkristallisation isoliert. Für viele Anwendungszwecke ist bereits die erzielte Reinheit des Rohproduktes befriedigend.

Die Reaktionsprodukte des erfindungsgemäßen Verfahrens sind am Stickstoff α-Methoxy-alkylierte Urethane der Formel (II)

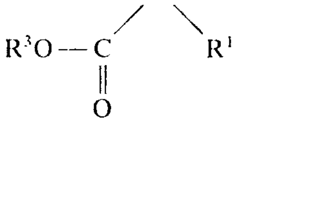

(II)

in der

$R^1$ bis $R^3$ die oben angegebene Bedeutung besitzen.

Bevorzugte Reaktionsprodukte sind solche der Formel (III)

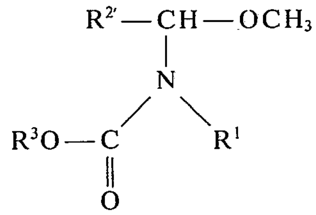

(III)

in der

$R^1$ für Wasserstoff, Alkyl, Alkenyl, $-CH_2-OCH_3$, $-CH(Alkyl)-OCH_3$ oder $-CH(Alkenyl)-OCH_3$ steht,
$R^{2'}$ Alkyl oder Alkenyl bedeutet und
$R^3$ für Alkyl, Alkenyl oder $\omega$-Hydroxy-alkyl steht,

wobei außerdem $R^1$ und $R^{2'}$ gemeinsam eine Alkylengruppe darstellen können, die mit den Atomen, die zwischen ihnen stehen, einen Ring bildet, und wobei außerdem $R^{2'}$ und $R^3$ gemeinsam eine Alkylengruppe darstellen können, die mit den Atomen, die zwischen ihnen stehen, einen Ring bildet.

Die Erfindung betrifft weiterhin die neuen am Stickstoff $\alpha$-Methoxy-alkylierten Urethane der Formel (IV)

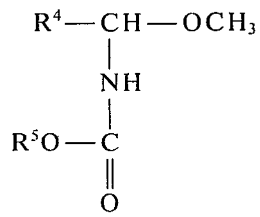

(IV)

in der

$R^4$ für $C_1-C_4$-Alkyl steht und
$R^5$ für geradkettiges oder verzweigtes $\omega$-Hydroxy-$C_1-C_4$-Alkyl steht.

Das erfindungsgemäße Verfahren ergibt die am Stickstoff $\alpha$-Methoxy-alkylierten Urethane in hohen Ausbeuten ohne Bildung schwer abtrennbarer Nebenprodukte bei gleichzeitig hoher Stromausbeute.

Die Erfindung stellt weiterhin einen Fortschritt durch die Bereitstellung der neuen Verbindungen der Formel (IV) dar.

Es ist als ausgesprochen überraschend zu bezeichnen, daß gegenüber den bekannten Verfahren keine durch Abspaltung von Substituenten gebildeten Nebenprodukte entstehen und daß auch O-($\omega$-Hydroxyalkyl)-urethane und cyclische Urethane der Methoxylierung zugänglich sind. Weiterhin ist als überraschend zu bezeichnen, daß im erfindungsgemäßen Verfahren ein nahezu 100%iger Umsetzungsgrad und dabei außerdem eine nahezu 100%ige Selektivität erreicht werden können, während beispielsweise bei der Oxidation von N-Alkyl-amiden eine Weiteroxidation des Mono-methoxylierten Amids mit der Oxidation des Ausgangsproduktes konkurriert (Acta Chem. Scand. B 32, 182 (1978)).

Die Reaktionsprodukte des erfindungsgemäßen Verfahrens, insbesondere die neuen am Stickstoff $\alpha$-Methoxy-alkylierten Urethane, können Anwendung als verkappte Formaldehydvernetzer (DE-A-2 223 484) oder als Ausgangsprodukte für Vinylurethane, die zur Herstellung von wertvollen Polymerprodukten (J. Polymer Sci., Part B, 7, 181 (1969); Copolymere mit Acrylamid nach FR-A-1 514 870; Copolymere mit Äthylen nach DE-B-1 247 022) geeignet sind, finden.

## Beispiel 1

300 g (3,37 Mol) O-Methyl-N-methyl-urethan, gelöst in 1,4 l Methanol, das 2 ml Collidin und 33 g Tetraäthylammonium-tetrafluoroborat ($Et_4NBF_4$) gelöst enthält, werden bei einer Temperatur von 22 bis 25°C an Platinanoden (Gesamtfläche 2,4 $dm^2$) in einer Elektrolyseapparatur bei einer Stromdichte von 12,5 $A/dm^2$ bis zum Verbrauch von 140 Ah, dann bei einer Stromdichte von 8 $A/dm^2$ bis zum Verbrauch von 40 Ah und bei einer Stromdichte von 4 $A/dm^2$ bis zum Verbrauch von 20 Ah, also insgesamt bis zum Verbrauch von 200 Ah = 2,2 F/Mol Ausgangsverbindung elektrolysiert. Die mittlere

Klemmenspannung beträgt 8,5 V. Man engt den Elektrolyten am Rotationsverdampfer ein, filtriert vom ausgefallenen Leitsalz ab und destilliert. Man erhält 365 g (3,06 Mol) O-Methyl-N-methoxymethyl-urethan $Kp_{0,3}$ : 45°C, das entspricht einer Materialausbeute von 91% und einer Stromausbeute von 82%, jeweils bezogen auf die theoretische Ausbeute.

Beispiel 2

515 g (5 Mol) O-Methyl-N-äthyl-urethan in 1,4 l Methanol, das 1,5 ml Collidin und 40 g $Et_4NBF_4$ enthält, werden, wie im Beispiel 1 beschrieben, bei einer Stromdichte von 12,5 A/dm² bis zum Verbrauch von 240 Ah, bei 8 A/dm² bzw. 4/dm² bis zum Verbrauch von 40 bzw. 20 Ah elektrolysiert. Die mittlere Klemmenspannung beträgt 9 V.Man isoliert destillativ 612 g (4,6 Mol) O-Methyl-N-(1-methoxyäthyl)-urethan mit einem $Kp_{12}$ : 70°C, entsprechend einer Materialausbeute von 92% und einer Stromausbeute von 89%, jeweils bezogen auf die theoretische Ausbeute.

Beispiel 3

198 g (1,92 Mol) O-Methyl-N,N-dimethyl-urethan, gelöst in 600 cm³ Methanol, 22 g $Et_4NBF_4$ und 2 ml Collidin werden wie in Beispiel 2 bei abnehmender Stromdichte bis zum Verbrauch von 240 Ah = 4,66 F/Mol elektrolysiert. Nach der üblichen Aufarbeitung erhält man durchfraktionierte Destillation 92 g (0,69 Mol) = 36% O-Methyl-N-methyl-N-methoxy-methyl-urethan $Kp_{0,15}$ : 55°C und 111 g (0,68 Mol) = 35% O-Methyl-N,N-dimethoxymethyl-urethan, $Kp_{0,15}$ : 63°C, jeweils bezogen auf die theoretische Ausbeute.

Beispiel 4

282 g (2,4 Mol) O,N-Diäthylurethan, gelöst in 1 l Methanol, 30 g $Et_4NBF_4$ und 0,5 ml Collidin werden wie in den Beispielen 1 bis 3 an Pt-Anoden bis zum Verbrauch von 2,48 F/Mol elektrolysiert. Die übliche Aufarbeitung ergibt 274 g (1,86 Mol) O-Äthyl-N-(1-methoxyäthyl)-urethan vom $Kp_{0,1}$ : 45°C, entsprechend einer Ausbeute von 78% der theoretischen Ausbeute.

Beispiel 5

424 g (3,56 Mol) O-(2-Hydroxyäthyl)-N-methyl-urethan, gelöst in 1,2 l $CH_3OH$, 33 g $Et_4NBF_4$ und 2 ml Collidin werden an Pt-Anoden bei 12,5 A/dm² bis zum Verbrauch von 200 Ah, bei 8 A/dm² bis zum Verbrauch von 45 Ah und bei 4 A/dm² bis zum Verbrauch von 40 Ah, (insgesamt = 285 Ah = 3 F/Mol) elektrolysiert.

Nach dem Abziehen des Lösungsmittels und dem Absaugen des ausgefallenen Leitsalzes isoliert man durch Dünnfilmdestillation 482 g O-(2-Hydroxyäthyl)-N-methoxymethyl-urethan, $Kp_{0,1}$ : 120°C, in 98%iger Reinheit. Das entspricht einer Ausbeute von 89% und einer Stromausbeute von 60%, jeweils bezogen auf die theoretische Ausbeute.

Beispiel 6

410 g (3,45 Mol) O-(2-Hydroxyäthyl)-N-methylurethan, gelöst in 1,2 l Methanol, 33 g $Et_4NBF_4$ und 1 ml Collidin werden an Graphit-Anoden bei 10 A/dm² bis zum Verbrauch von 180 Ah, bei 6 A/dm² bis zum Verbrauch von 40 Ah und bei 3 A/dm² bis zum Verbrauch von 40 Ah (insgesamt = 260 Ah = 2,8 F/Mol) elektrolysiert. Man isoliert, wie im Beispiel 5 beschrieben, 464 g Produkt in 94%iger Reinheit, das noch 6% Ausgangsprodukt enthält, entsprechend einer Ausbeute von 85% der theoretischen Ausbeute.

Beispiel 7

399 g (3 Mol) O-(2-Hydroxyäthyl)-N-äthylurethan, gelöst in 1,3 l Methanol, 1 ml Collidin und 33 g $Et_4NBF_4$ werden an Pt-Anoden bei 12,5 A/dm² bis zum Verbrauch von 180 Ah und bei 4 A/dm² bis zum Verbrauch von 30 Ah (insgesamt = 210 A = 2,6 F/Mol) elektrolysiert. Nach dem Abdestillieren des Lösungsmittels und Absaugen des Leitsalzes erhält man 493 g Rohprodukt, das durch Dünnfilmdestillation gereinigt wird und 445 g = 91% der theoretischen Ausbeute O-(2-Hydroxyäthyl)-N-(1-methoxyäthyl)-urethan ergibt, dessen Reinheit durch NMR-Spektroskopie festgestellt wurde.

Beispiel 8

348 g (4 Mol) Oxazolidinon-(2), gelöst in 1,2 l Methanol,30 g $Et_4NBF_4$ und 3 ml Collidin werden an Pt-Anoden bei 12,5 A/dm $^2$ bis zum Verbrauch von 180 Ah, bei 8 A/dm$^2$ bis zum Verbrauch von 40 Ah und bei 4 A/dm$^2$ bis zum Verbrauch von 20 Ah (insgesamt = 240 Ah = 2,24 F/Mol) oxidiert. Nach dem Abdestillieren von Methanol wird der Rückstand aus Essigester umkristallisiert. Man erhält 322 g (69% der theoretischen Ausbeute) 4-Methoxy-oxazolidinon-(2), Fp: 65 bis 66° C. Weiteres Produkt ist neben dem Leitsalz in der Mutterlauge enthalten.

Beispiel 9

748 g (4 Mol) O-(2-Äthylhexyl)-N-methylurethan, gelöst in 2 l $CH_3OH$, 50 g $Et_4NBF_4$ und 3 ml Collidin werden an Pt-Anoden bei 12,5 A/dm$^2$ bis zum Verbrauch von 120 Ah und bei 8 A/dm$^2$ bzw. 4 A/dm$^2$ bis zum Verbrauch von 80 Ah bzw. 40 Ah elektrolysiert. Nach der üblichen Aufarbeitung isoliert man destillativ ($Kp_{0,3}$ : 102° C) 798 g (3,68 Mol) O-(2-Äthylhexyl)-N-methoxymethyl-urethan, entsprechend einer Ausbeute von 92% der theoretischen Ausbeute.

Beispiel 10

Man löst 345 g (3 Mol) O-Allyl-N-methyl-urethan in 1,5 l Methanol, das 40 g Tetraäthylammoniumtetrafluoroborat und 1 ml Collidin gelöst enthält, und elektrolysiert an Platin-Anoden bei Stromdichten von 12,5 bzw. 8 bzw. 4 A/dm$^2$ bis zum Verbrauch von 120 bzw. 40 bzw. 20 Ah. Das entspricht einer Gesamtstrommenge von 180 Ah bzw. 2,24 Faraday pro Mol. Nach der Aufarbeitung wie in Beispiel 1 werden durch Vakuumdestillation 359 g (= 82% der theoretischen Ausbeute) O-Allyl-N-methoxymethyl-urethan mit einem $Kp_{0,2}$ : 79° C erhalten.

Beispiel 11

618 g (6 Mol) O-Methyl-N-äthyl-urethan werden in 2 l Methanol gelöst, das 46 g Tetraäthylammonium-tetrafluoroborat, jedoch keine basische Verbindung, gelöst enthält. Der Elektrolyt wird an Platin-Anoden bei 12 A/dm$^2$ Stromdichte bis zum Verbrauch von 300 Ah, bei 8 A/dm$^2$ bis zum Verbrauch von weiteren 40 Ah und bei 4 A/dm$^2$ bis zum Verbrauch von weiteren 20 Ah elektrolysiert. Das bedeutet einen Gesamtverbrauch von 360 Ah bzw. 2,24 Faraday pro Mol. Nach dem Abdestillieren des Lösungsmittels im Vakuum fallen 46 g Leitsalz kristallin aus. Man erhält 774 g Rohprodukt, das durch Vakuumdestillation gereinigt wird. Als Reinprodukt erhält man 706 g O-Methyl-N-methoxymethylurethan vom $Kp_{0,2}$ : 52° C (entspricht 76% der theoretischen Ausbeute).

**Patentansprüche**

1. Verfahren zur Herstellung von am Stickstoff $\alpha$-Methoxyalkylierten Urethanen durch elektrochemische Oxidation von am Stickstoff alkylierten Urethanen in methanolischer Lösung in Gegenwart eines Leitsalzes bei einer Temperatur unter bis weit über 0° C, einer Strommenge von mindestens 2 Faraday pro Mol eingeführte Methoxygruppe und einer Stromdichte bis zu 50 A/dm$^2$, dadurch gekennzeichnet, daß man die Elektrolyse im Temperaturbereich von −20 bis +65° C bei einer Stromdichte von 0,1 bis 50 A/dm$^2$ durchführt, wobei die Stromdichte im Verlauf der Elektrolyse erniedrigt wird, und daß man gegebenenfalls in Gegenwart einer basischen Verbindung arbeitet.

2. Verfahren nach Anspruch 1, dadurch gekennzeichnet, daß man Urethane der Formel

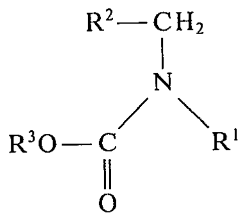

in der

$R^1$ für Wasserstoff, Alkyl, Alkenyl, $-CH_2-OCH_3$, $-CH(Alkyl)-OCH_3$ oder $-CH(Alkenyl)-OCH_3$ steht,

$R^2$ Wasserstoff, Alkyl oder Alkenyl bedeutet und
$R^3$ für Alkyl, Alkenyl oder $\omega$-Hydroxy-alkyl steht,

wobei außerdem $R^1$ und $R^2$ gemeinsam eine Alkylengruppe darstellen können, die mit den Atomen, die zwischen ihnen stehen, einen Ring bildet, und wobei außerdem $R^2$ und $R^3$ gemeinsam eine Alkylengruppe darstellen können, die mit den Atomen, die zwischen ihnen stehen, einen Ring bildet, in methanolischer Lösung, die 1 bis 50 Gew.-% Urethan enthält, einsetzt.

3. Verfahren nach Anspruch 1, dadurch gekennzeichnet, daß als Leitsalz Tetraäthylammonium-tetrafluorborat in einer Konzentration von 0,01 bis 2 Mol pro Liter Elektrolyt eingesetzt wird.

4.Verfahren nach Anspruch 1, dadurch gekennzeichnet, daß als Anodenmaterial Platin oder platiniertes Titan verwendet wird.

5. Am Stickstoff $\alpha$-Methoxy-alkylierte Urethane der Formel

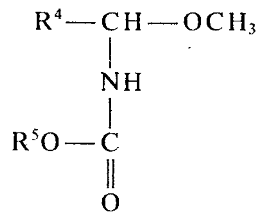

in der

$R^4$ für $C_1-C_4$-Alkyl steht und
$R^5$ für geradkettiges oder verzweigtes $\omega$-Hydroxy-$C_1-C_4$-Alkyl steht.

**Claims**

1. Process for the preparation of urethanes which are $\alpha$-methoxy-alkylated on the nitrogen, by electrochemical oxidation of urethanes which are alkylated on the nitrogen, at a temperature of below to far higher than 0°C, using an amount of current of at least 2 farads per mol of methoxy group introduced and at a current density of up to 50 A/dm², characterised in that the electrolysis is carried out in a temperature range of $-20$ to $+65$°C and at a current density of 0.1 to 50 A/dm², the current density being reduced in the course of the electrolysis, and in that the process ist optionally conducted in the presence of a basic compound.

2. Process according to Claim 1, characterised in that urethanes of the formula

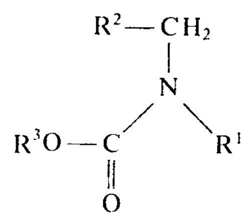

in which

$R^1$ represents hydrogen, alkyl, alkenyl, $-CH_2-OCH_3$, $-CH(alkyl)-OCH_3$ or $-CH(alkenyl)-OCH_3$,
$R^2$ denotes hydrogen, alkyl or alkenyl and
$R^3$ represents alkyl, alkenyl or $\omega$-hydroxy-alkyl,

and wherein

$R^1$ and $R^2$ together can also represent an alkylene group, which forms a ring with the atoms between $R^1$ and $R^2$,

and wherein

$R^2$ and $R^3$ together can also represent an alkylene group, which forms a ring with the atoms between $R^2$ and $R^3$,

in methanolic solution which contains 1 to 50% by weight of urethane are employed.

3. Process according to Claim 1, characterised in that tetraethylammonium tetrafluoborate is employed as the conducting salt in a concentration of 0.01 to 2 mols per litre of electrolyte.

4. Process according to Claim 1, characterised in that platinum or platinised titanium is used as the anode material.

5. Urethanes which ar $\alpha$-methoxy-alkylated on the nitrogen, of the formula

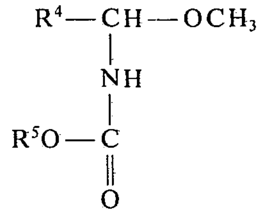

in which

$R^4$ represents $C_1-C_4$-alkyl and
$R^5$ represents straight-chain or branched -hydroxy-$C_1-C_4$-alkyl.

**Revendications**

1. Procédé pour la préparation d'uréthannes $\alpha$-méthoxy-alkylés à l'azote par oxydation électrochimique d'uréthannes alkylés à l'azote en solution méthanolique en présence d'un sel conducteur à une température inférieure jusqu' à très supérieure à 0° C, à une quantité de courant d'au moins 2 farads par mole de groupe méthoxy introduit et une densité de courant allant jusqu'à 50 A/dm², caractérisé en ce que l'on effectue l'électrolyse dans le domaine de température de −20 à +65° C avec une densité de courant de 0,1 à 50 A/dm², la densité de courant étant abaissée au cours de l'électrolyse, et en ce que l'on opère éventuellement en présence d'un composé basique.

2. Procédé selon la revendication 1,caractérisé en ce que l'on utilise des uréthannes de formule

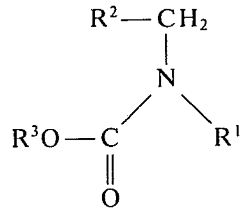

dans laquelle

$R^1$ représente l'hydrogène, un alkyle, un alcényle, $-CH_2-OCH_3$, $-CH(alkyl)-OCH_3$ ou $-CH(alcényl)-OCH_3$,
$R^2$ représente l'hydrogène, un alkyle ou un alcényle, et
$R^3$ représente un alkyle, un alcényle ou un $\omega$-hydroxyalkyle,

$R^1$ et $R^2$ pouvant en outre ensemble former un groupe alkylène qui, avec les atomes placés entre eux, forme un cycle et $R^2$ et $R^3$ pouvant aussi former ensemble un groupe alkylène qui, avec les atomes placés entre eux, forme un cycle, dans une solution méthanolique qui contient 1 à 50% en poids d'uréthanne.

3. Procédé selon la revendication 1, caractérisé en ce que l'on utilise comme sel conducteur le tétrafluoroborate de tétraéthylammonium à une concentration de 0,01 à 2 moles par litre d'électrolyte.

4. Procédé selon la revendication 1, caractérisé en ce que l'on utilise comme matériau d'anode le platine ou le titane platiné.

5. Uréthannes $\alpha$-méthoxyalkylés à l'azote de formule

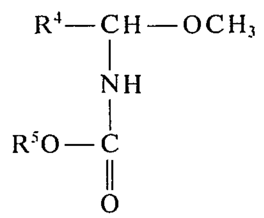

dans laquelle

$R^4$ représente un alkyle en $C_1-C_4$, et
$R^5$ représente un $\omega$-hydroxyalkyle en $C_1-C_4$ à chaîne droite ou ramifiée.